(19) Europäisches Patentamt
European Patent Office
Office européen des brevets

(11) Publication number: **0 164 922**
**B1**

(12) # EUROPEAN PATENT SPECIFICATION

(45) Date of publication of patent specification: **30.08.89**

(51) Int. Cl.⁴: **C 07 C 67/00**, C 07 C 69/14, C 07 C 51/083, C 07 C 53/08

(21) Application number: **85303438.7**

(22) Date of filing: **16.05.85**

(54) **Hydrogenation process.**

(30) Priority: **22.05.84 GB 8413117**
**16.10.84 GB 8426100**

(43) Date of publication of application:
**18.12.85 Bulletin 85/51**

(45) Publication of the grant of the patent:
**30.08.89 Bulletin 89/35**

(84) Designated Contracting States:
**AT BE DE FR GB IT LU NL SE**

(56) References cited:
**EP-A-0 061 395**
**US-A-4 221 918**
**US-A-4 374 265**

(73) Proprietor: **BP Chemicals Limited**
**Belgrave House 76 Buckingham Palace Road**
**London, SW1W 0SU (GB)**

(72) Inventor: **Williams, Peter Sefton**
**BP Chemicals Limited Saltend**
**Hedon Hull HU12 8DS (GB)**

(74) Representative: **Fawcett, Richard Fennelly et al**
**BP INTERNATIONAL LIMITED Patents Division**
**Chertsey Road**
**Sunbury-on-Thames Middlesex, TW16 7LN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 164 922 B1

Courier Press, Leamington Spa, England.

## Description

The present invention relates to the hydrogenation of carboxylic acids, carboxylic acid anhydrides, and alkylidene dicarboxylates. In particular the present invention relates to such a hydrogenation process carried out in the presence of a rhodium metal catalyst.

US 4,398,039 discloses a process for the vapour phase hydrogenation of carboxylic acids to yield the corresponding acid or its ester. The catalyst used is a mixed oxide based on ruthenium modified with (i) zinc and/or cadmium, (ii) cobalt and/or nickel and (iii) mixtures of iron, copper, rhodium, palladium, osmium, iridium and platinum.

Our UK patent application No. 8328140 describes a process for the production of esters, e.g. ethyl acetate, by the hydrogenation of an acid anhydride, e.g. acetic anhydride, in the presence of a Group VIII metal, for example ruthenium. However, in this application the reaction is carried out in the liquid phase and the Group VIII metal is present as a soluble inorganic or organometallic compound and not as the metal itself.

US—A—4,221,918 discloses a heterogeneous process for preparing 1,1-diesters which comprises contacting a carboxylic acid anhydride with hydrogen in the presence of an insoluble metal hydrogenation catalyst and a strong protonic acid having a PKa of less that 4, said carboxylic acid anhydride being selected from the group consisting of anhydrides having the structure:—

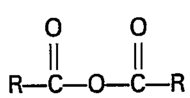

wherein R is an alkyl group having from 1 to 20 carbon atoms, a cycloalkyl group having from 5 to 8 carbon atoms, an alkenyl or alkynyl group having from 2 to 20 carbon atoms, aryl or alkaryl groups having from 6 to 12 carbon atoms, or aralkyl groups having from 7 to 12 carbon atoms. Palladium on charcoal is the preferred catalyst.

It has now been discovered that the hydrogenation of carboxylic acid anhydrides, alkylidene dicarboxylates or mixtures thereof can be effected using a rhodium catalyst, where the rhodium is present in the metallic state, and a strong acid promoter which is an acid stronger than acetic acid.

Accordingly, the present invention provides a process for the hydrogenation of a feedback comprising a carboxylic acid anhydride, an alkylidene dicarboxylate, or a mixture thereof which process comprises reacting the feedstock with hydrogen at elevated temperature and pressure in the presence of, as catalyst, (a) rhodium in the metallic state and (b) a strong acid promoter which is an acid stronger than acetic acid.

The feedstock used in the hydrogenation process is suitably a carboxylic acid anhydride or an alkylidene dicarboxylate. Although in principle any such feedstock can be used, it is preferable to use a carboxylic acid anhydride derived from a $C_2$—$C_{10}$ carboxylic acid or an alkylidene dicarboxylate derived from a $C_2$—$C_{10}$ carboxylic acid and an alkylidene radical having less than 10 carbon atoms. Examples of preferred feedstocks include acetic anhydride, propionic anhydride, ethylidene diacetate and the like.

The products of the hydrogenation depend on the feedstock used. When the feedstock used is a carboxylic acid anhydride the hydrogenation product can be one or more of an alcohol, an ester of an alcohol and an alkylidene dicarboxylate while when an alkylidene dicarboxylate is used as feedstock an alcohol and/or an ester of an alcohol can be formed.

The rhodium used as the first component of the catalyst is present as the metal as opposed to a soluble inorganic complex of a higher oxidation state. It may be used supported or unsupported. When used unsupported the rhodium is preferably added in the reaction mixture in a finely divided form, for example rhodium black.

Although the rhodium metal may be added unsupported, it is in practice more convenient and preferable to support the rhodium metal on an inert solid since this allows the dispersion of the rhodium metal and hence the active surface area of the catalyst to be increased. By this means, a more efficient use is made of the rhodium metal.

The term inert solid is defined as a solid which (1) is not chemically or thermally degraded under the reaction conditions and (2) does not react with the feedstock in the absence of the rhodium metal catalyst. Typical supports which may be used to advantage will be familiar to those skilled in the art and include, carbons, graphites, silicas, aluminas, zeolites, aluminosilicates, diatomaceous earths and clays. Of these carbons, graphites and aluminas are preferred supports. Methods of preparing such catalysts will also be familiar to the skilled man. A preferred method however is one in which the support is impregnated with a rhodium salt or complex from aqueous solution, dried, and subsequently treated with hydrogen at elevated temperature to generate the rhodium metal in the metallic state on the support. Conveniently, the supported catalyst is prepared so as to contain less than 25% by weight rhodium metal.

In addition to the rhodium metal, there is added a second catalyst component comprising a strong acid, which is an acid stronger than acetic acid (i.e. its pKa at 25% is less than 4.75) and is chemically inert under the reaction conditions as promoter. Thus the acid can be a strong mineral acid e.g. phosphoric acid, a fluorinated carboxylic acid, e.g. trifluoracetic acid or a sulphonic acid. Preferred sulphonic acids include methanesulphonic acid, trifluormethylsulphonic acid, benzenesulphonic acid and p-toluenesulphonic acid. It is also possible to use solid polymeric sulphonic acids, for example polyperfluorosulphonic acids such as those sold

under the trade name "Nafion", admixed with the Group VIII metal catalyst or as a support for the Group VIII metal itself.

The strong acid component is conveniently added in amounts up to 10% by weight of the total reactor charge.

The reaction is conveniently carried out at temperatures in excess of 50°C, preferably in the range of 75 to 250°C. It will be appreciated that for each feedstock the optimum reaction temperature will be different but in general it is desirable to carry out the process at the lowest temperature possible in order to minimise the formation of alkane byproducts.

The hydrogen gas used to effect the hydrogenation is conveniently fed to the reactor at superatmospheric pressure, preferably in the range 250 to 5,000 psi. It may contain impurities such as carbon monoxide, nitrogen and the inert gases e.g. helium.

The hydrogenation process may be suitably carried out in either the liquid phase or the vapour phase. If carried out in the liquid phase, the rhodium metal catalyst is suspended in the liquid feedstock and the promoter is either dissolved or suspended in the feedstock also. An organic solvent which is inert under the reaction conditions can also be added if so desired although this is not essential if both the feedstock and its hydrogenation products are liquid at room temperature.

As an alternative to carrying out the hydrogenation in the liquid phase, it is also possible to effect the hydrogenation by a vapour phase process wherein the reactants, in the vapour phase, and hydrogen are fed to a reactor containing a bed of the solid catalyst. In either case the process can be carried out batchwise or continuously.

The present invention will now be illustrated by reference to the following Example.

Example

This example demonstrates that using rhodium as catalyst both carboxylic acid esters and alkylidene dicarboxylates can be obtained.

To an autoclave was added 150.1 g acetic anhydride, 1.5 g methanesulphonic acid, and 5.59 g rhodium metal on carbon in a paste form containing *ca* 50% water (metal loading on dry powder 5% wt). The autoclave was purged with hydrogen, then pressurised to 1420 psig with hydrogen, before being heated with rapid stirring. When the autoclave reached *ca* 100°C, a very rapid reaction began and the temperature rose rapidly to 168°C. During the reaction period the pressure was maintained at 1300±200 psig. After completion of the reaction (estimated time *ca* 0.75 hours) the autoclave was cooled and the product analysed by gas chromatography. The following composition was found:

| | |
|---|---|
| ethyl acetate | 8.2% wt |
| ethylidene diacetate | 20.7% wt |
| acetaldehyde | 1.5% wt |
| acetic acid | 61.3% wt |

**Claims**

1. A process for the hydrogenation of a feedstock comprising a carboxylic acid anhydride, an alkylidene dicarboxylate or a mixture thereof which process comprises reacting the feedstock with hydrogen at elevated temperature and pressure in the presence of, as catalyst, (a) rhodium in the metallic state and (b) a strong acid which is an acid stronger than acetic acid.

2. A process according to claim 1 wherein the strong acid (b) is a fluorinated carboxylic acid.

3. A process according to claim 2 wherein the fluorinated carboxylic acid is trifluoroacetic acid.

4. A process according to claim 1 wherein the strong acid (b) is a sulphonic acid which is either methane sulphonic acid, trifluoromethanesulphonic acid, benzenesulphonic acid or p-toluenesulphonic acid.

5. A process according to claim 1 wherein the strong acid (b) is a polyperfluorosulphonic acid.

6. A process according to claim 1 wherein the rhodium metal is supported on an inert solid support.

7. A process according to any one of the preceding claims wherein the feedstock is acetic anhydride and the product comprises ethyl acetate and ethylidene diacetate.

**Patentansprüche**

1. Verfahren zum Hydrieren eines ein Carbonsäureanhydrid, eine Alkylidendicarboxylat oder ein Gemisch hievon enthaltenden Einsatzmaterials, welches Verfahren ein Umsetzen des Einsatzmaterials mit Wasserstoff bei erhöhter Temperatur und erhöhtem Druck in Gegenwert von (a) Rhodium in metallischem Zustand und (b) einer starken Säure, die stärker ist als Essigsäure, als Katalysator umfaßt.

2. Verfahren nach Anspruch 1, worin die starke Säure (b) eine fluorierte Carbonsäure ist.

3. Verfahren nach Anspruch 2, worin die fluorierte Carbonsäure Trifluoressigsäure ist.

4. Verfahren nach Anspruch 1, worin die starke Säure (b) eine Sulfonsäure ist, die entweder Methansulfonsäure, Trifluormethansulfonsäure, Benzolsulfonsäure oder p-Toluolsulfonsäure ist.

5. Verfahren nach Anspruch 1, worin die starke Säure (b) eine Polyperfluorsulfonsäure ist.

6. Verfahren nach Anspruch 1, worin das metallische Rhodium auf einem inerten festen Träger aufgebracht ist.

7. Verfahren nach einem der vorstehenden Ansprüche, worin das Einsatzmaterial Essigsäureanhydrid ist und das Produkt Ethylacetat und Ethylidendiacetat enthält.

**Revendications**

1. Procédé pour l'hydrogénation d'une charge d'alimentation, comprenant un anhydride d'acide carboxylique, un dicarboxylate d'alkylidène ou un de leurs mélanges, ce procédé comprenant la mise en réaction de la charge d'alimentation avec de l'hydrogène, à température et sous une pression élevées, en présence, comme catalyseur, (a) de rhodium à l'état métallique et (b) d'un acide fort, qui est un acide plus fort que l'acide acétique.

2. Procédé selon la revendication 1, dans lequel l'acide fort (b) est un acide carboxylique fluoré.

3. Procédé selon la revendication 2, dans lequel l'acide carboxylique fluoré est l'acide trifluoracétique.

4. Procédé selon la revendication 1, dans lequel l'acide fort (b) est un acide sulfonique, qui est l'acide méthanesulfonique, l'acide trifluorométhanesulfonique, l'acide benzènesulfonique ou l'acide p-toluènesulfonique.

5. Procédé selon la revendication 1, dans lequel l'acide fort (b) est un acide polyperfluorosulfonique.

6. Procédé selon la revendication 1, dans lequel le rhodium métallique est un support solide inerte.

7. Procédé selon l'une quelconque des revendications précédentes, dans lequel la charge d'alimentation est de l'anhydride acétique, et le produit obtenu comprend de l'acétate d'éthyle et du diacétate d'éthylidène.